# EUROPEAN PATENT APPLICATION

(11) **EP 4 506 446 A1**
(43) Date of publication of application: **12.02.2025**
(21) Application number: 23784400.6
(22) Date of filing: 29.03.2023
(51) Int. Cl.: C12N 5/077, A61K 35/28

(54) **METHOD FOR ENHANCING THE OSTEOGENIC DIFFERENTIATION OF MESENCHYMAL STROMAL CELLS**

(30) Priority: 07.04.2022 ES 202230314
(71) Applicant: Universidad Complutense De Madrid, 28040 Madrid (ES)
(72) Inventor: ZAPATA GONZÁLEZ, Agustín Gregorio, 28047 Madrid (ES); ALFARO SÁNCHEZ, David, 28047 Madrid (ES); RODRÍGUEZ SOSA, Mariano Rubén, 28004 Madrid (ES)
(74) Representative: ABG Intellectual Property Law, S.L.
(86) International application number: PCT/ES2023/070199
(87) International publication number: WO 2023/194637

(57) **Abstract**

The invention relates to a method for enhancing the osteogenic differentiation of mesenchymal stromal cells (MSCs) by culturing same in an osteogenic medium with an added EphB3-receptor blocking reagent. The blocking reagent may be selected from polyclonal antibodies, monoclonal antibodies, fusion proteins or a combination thereof. The invention also relates to the cells of the osteogenic lineage obtained using the described method and to the EphB3-receptor blocking reagents, for use in the treatment of bone diseases causing bony tissue deficiency and/or bone fractures. The invention also relates to pharmaceutical compositions containing the cells of the osteogenic lineage obtained using the method and/or the EphB3-receptor blocking reagents.

## Description

### FIELD OF THE ART

The present invention is comprised in the sector of pharmaceutical products. More specifically, it relates to obtaining bony tissue from the mesenchymal stromal cells of animals and, particularly, mammals, and to methods, reagents, compounds, and/or molecules for use in the treatment of bone diseases and/or fractures.

### BACKGROUND OF THE INVENTION

Every year more than 20 million patients around the world are affected by the loss of bony tissue caused by disease or trauma. More than half a million interventions related to bone defects were performed in the United States by 2005, with a cost of more than $20.5 billion, while the cost in the European Union in 2010 was estimated at €37 billion, and is expected to increase by 25% for 2025.

Along with trauma, bone deficit problems are often associated with several factors, such as age, sex, and infections, as evidenced by diagnoses of osteoporosis, osteopenia, and severe dental problems related to tooth loss. Functional deterioration of mesenchymal stromal cells (MSCs) and osteoblast progenitor cells has also been proposed as a pathological mechanism contributing to bone disorders such as osteoporosis (the most common bone disease) and other rare inherited skeletal dysplasias, which may be due not only to increased bone resorption, but also to problems in the osteogenic differentiation of their progenitors, including MSCs.

Osteoporosis has been recognized as one of the most important diseases that affect the world's population, along with hypertension and diabetes mellitus, and its economic impact is similar to the cost of other important diseases such as stroke, breast cancer, or myocardial infarction.

In the case of osteoporosis, its treatment seeks to maintain bone mass at normal levels. Current therapies are mainly drugs that inhibit bone resorption or improve osteogenesis.

Bisphosphonates are the most widely used reagents for the treatment of osteoporosis and act on osteoclasts, inhibiting bone resorption. Denosumab, an anti-RANKL (receptor activator of nuclear factor-kB ligand) antibody is also an antiresorptive agent. There are few bone anabolic agents: teriparatide, a parathyroid hormone analog that induces bone formation, and romosozumab, an anti-sclerostin antibody that improves bone formation and inhibits resorption. However, most antiresorptive treatments exhibited side effects and bone anabolic products are few and far between. Therefore, there is an urgent need to develop new osteoanabolic therapies to promote bone formation (Hu et al. Int. J. Mol. Sci. 2018, 19, 360; doi:10.3390/ijms19020360).

With the rapid development of cell therapy, human MSCs emerge as a suitable tool to study the etiology of bone disorders at the cellular level, and to be used therapeutically for bone diseases, among many other pathologies.

MSCs are multipotent progenitors that exist in almost all organs and tissues, such as bone marrow, adipose tissue, liver, umbilical cord, muscles, and tooth pulp. MSCs are plastic-adherent cells, capable of self-renewal and differentiation into multiple cell lineages, mainly derived from the medial leaflet, such as osteoblasts, adipocytes, and chondrocytes. Differentiation into a bone lineage is modulated by several internal and external factors that modulate this process. With respect to internal factors, several molecules which regulate MSC bone differentiation have been described: Runx2, Sp7 (Osterix), TAZ, Foxc2, Twist, BMP, Wnt/β-catenin, IGF-1, miR-21, miR-31, miR-138, miR-204, miR-637, etc., although there are also other external factors such as: mechanical stimuli (exercise, vibration), radiation, and high-fat diet (Hu et al. Int. J. Mol. Sci. 2018, 19, 360; doi:10.3390/ijms19020360).

One of the most relevant factors is BMP-2, which has been used alone, in the form of recombinant protein, and together with other factors such as: vitamin D, dexamethasone, PDGF, PGF, BMP-7, NELL-1, Ang-1, WSIP1, or PRP. The use of BMP-2 has been shown to be effective in several tests such as in periodontal regeneration, in some face grafts, and in "non-unions" in oral surgery. Other such factors like bFGF, PDGF-BB, HGF, or IGF-1 have been overexpressed in MSCs, with an increase in osteogenic differentiation also being observed. (Kim S et al. Adv Exp Med Biol. 2018; 1078:233-244. doi: 10.1007/978-981-13-0950-2_12). Similarly, platelet lysates accompanied by factors such as FGF, TGF, IGF, VEGF, PDGF, IGF, BMP-2, tyrosine, calcitrol, stonozolol, dexamethasone, β-glycerophosphate, and/or F-ascorbic acid have been used (document WO2020161748A1).

However, at present, most *in vivo* bone regeneration tests simply use isolated and cultured autologous or allogeneic MSCs, without any modification that improve their effectiveness.

Another candidate currently being studied as a regulator of MSC behavior is the Eph/Ephrin system. Ephs (erythropoietin-producing hepatocellular receptors) are the largest family of tyrosine kinase receptors existing in eukaryotic cells and are involved in processes of cell attraction/repulsion, adhesion, survival, proliferation, and differentiation by means of interaction with their ligands Ephrins.

Ephrins are divided into two families according to their gene sequence and molecular structure: Ephrins A are proteins with a membrane-bound GPI (glycosylphosphatidylinositol) domain, whereas Ephrins B have a transmembrane region, followed by a cytoplasmic fragment with a PDZ domain. Ephs are also divided into two groups, A and B, based on their sequence homology and different affinity for the two types of Ephrins. These molecules performs bidirectional signaling: Ephs transmit a signal, called forward signal, into the cell where they are expressed, whereas Ephrins transmit signals called reverse signals.

Since their discovery three decades ago, the involvement of Ephs and Ephrins has been described in an increasing number of physiological and pathological processes in many cell types and different organs. Eph/Ephrin signaling is involved in multiple cellular processes such as: actin cytoskeleton remodeling, cell-substrate adhesion, intercellular junctions, cell shape, and cell movement. Eph/Ephrin signaling pathways are therefore determinant factors in processes such as cell migration and proliferation, cell distribution and positioning, cell commitment and differentiation, tissue patterning and morphogenesis, angiogenesis, and tissue plasticity. Furthermore, their involvement in cell survival, immune function, secretion, and tissue repair after injury has also been described (Niethamer, T. K., & Bush, J. O. (2019). Getting direction(s): The Eph/ephrin signaling system in cell positioning. Developmental Biology, 447(1), 42-57. doi: 10.1016/j.ydbio.2018.01.012).

Specifically, the relevance of Eph/Ephrins in bone repair is summarized as follows: EphrinB2 mediates osteogenesis in calvarial repair; EphB4 overexpression in osteoblasts increases bone formation in fracture callus during healing; EphrinB1-EphB2 forward and reverse signaling regulates a periosteal reaction to mechanical stress in bone; it has been proven that EphrinB2 and EphB4 modulate bone formation and resorption by activating osteoclasts or osteoblasts, respectively (document US20090186026A1). Recently, Eph/Ephrin expression during bone callus formation in fractures has been proven to be restricted to EphA4, EphA5, and EphrinB1, with EphrinB1 and EphA5 being observed as the main mediators of chondrocyte hypertrophy and ossification, respectively (Kaur et al. Changes in ephrin gene expression during bone healing identify a restricted repertoire of ephrins mediating fracture repair. Histochemistry and Cell Biology (2019) 151:43-55). In 2021, Kamath and Benson have published a paper with conflicting results on the involvement of EphB3 as a potential mediator of osteogenesis in both the development and repair of injuries (Kamath RAD, Benson MD. EphB3 as a potential mediator of developmental and reparative osteogenesis. Cells Tissues Organs. 25 October 2021. doi: 10.1159/000520369. Epub ahead of print. PMID: 34695818.). The few results described are discordant as they seem to depend on the bone parameter studied and the age of the animals analyzed. Furthermore, in the discussion, the authors indicate, on one hand, that EphB3 could be a candidate receptor for the action of Ephrins on developing bone growth and, on the other hand, that EphB3 functions by limiting rather than promoting osteogenesis. They attribute these contradictory results to the difficulty of assessing the effect of a single EphB receptor due to the functional redundancy exhibited by this family of molecules.

Furthermore, *in vitro* activation of EphB2 with a fusion protein causes increased osteogenesis in human MSCs originating from bone marrow. In mice, deletion of EphrinB1 in collagen 1α2-producing cells causes unsuccessful bone development, whereas activation of EphrinB1 reverse signal is essential for bone marrow stromal cell differentiation and bone formation. In this line, EphB2 forward signal and EphrinB1 reverse signal have been described as necessary for the mineralization of the extracellular matrix of the tooth pulp. Moreover, the EphB4/EphrinB2 pair, which is involved in hematopoietic precursor maturation and differentiation, also affects the osteogenic capacity of the bone marrow stroma, such that the absence of EphB4 causes alterations in the bone marrow, increasing the amount of bone precursors.

Therefore, there is significant interest in finding treatments that promote bone formation for addressing traumas and osteodegenerative diseases.

### DESCRIPTION OF THE INVENTION

Method for enhancing the osteogenic differentiation of mesenchymal stromal cells.

By definition, MSCs are capable of differentiating into an osteogenic lineage, although the mechanisms involved are barely known. Among all the elements that have been studied up until now as osteogenesis mediators, the Eph/Ephrin system is a possible candidate as it is a system involved in the communication between osteoblasts and osteoclasts, which mediates the duality between bone formation and bone remodeling, in a normal context of homeostasis. As set forth in the state of the art, among these molecules, some with osteoclastic effect and others attributed with osteoblastic effect are known: EphrinB1 deletion in osteoclast precursors promotes osteoclast development and function; EphA4 inhibits osteoclastic activity, but not osteoclast formation; EphrinB2 exerts osteoblastic effect by means of osteoblast EphB4 receptor activation; EphrinB1 is also capable of promoting osteoblastic differentiation, together with EphB1 and EphB2 receptors; ambivalent functions have been proposed for EphB3 receptor. However, in the case of the present invention, very solid and surprisingly positive results have been obtained with the EphB3 receptor. Specifically, high levels of osteogenic differentiation were obtained by blocking EphB3 signal. It was known up until now that EphB3 blocking is medically useful against different types of cancer such as liver carcinoma and colorectal carcinoma, and it can also be used in methods which confer neuroprotection and/or in treatments of tissue damage caused by ischemic events. However, in the studies on the Eph/Ephrin system and the bone system, this receptor has been repeatedly dismissed or at least little studied.

One aspect of the present invention relates to a method for enhancing the osteogenic differentiation of mesenchymal stromal cells including the use of a element capable of suppressing EphB3-mediated forward signaling in the mesenchymal stromal cells of mammals, including the human species. This absence of forward signaling favors the osteogenic differentiation of MSCs with the subsequent increase in bone matrix formation.

To perform this method for enhancing the osteogenic differentiation of mesenchymal stromal cells, it is advisable to first increase the number of MSCs to work with. To that end, the method begins with isolated MSCs which are cultivated *in vitro* to reach 80-90% confluence between the cells. After reaching that degree of confluence, the cells are lifted (detached) from the support where they have grown and incubated on a new support (or on several supports, depending on needs), using a conventional culture medium until the cells reach 80-90% confluence again (a process known as "passage"). The detachment and incubation are repeated as described above to increase the amount of MSCs (expansion phase) up to a maximum of six passages.

Isolated MSCs can be obtained from bone marrow or adipose tissue. To achieve improved MSC enrichment, the isolated cells can be cultured in culture media specific for this cellular type, which culture media favor MSC proliferation in comparison with any other cell type present after the isolation. Preferably, the culture medium is renewed at least once every 7 days in the different passages.

Once the desired amount of MSCs is obtained, the method for enhancing the osteogenic differentiation of MSCs includes the following steps:
1) after reaching 80-90% confluence level, replacing the culture medium used in the MSC expansion phase with an osteogenic culture medium and adding an EphB3-receptor blocking reagent;
2) incubating for at least 7 days.

Preferably, MSC differentiation is induced when a cell confluence of at least 90% has been achieved in culture. In this specification, confluence is understood to mean the percentage of occupation of the cells with respect to the surface of the support where they are being cultured, where confluence is considered to be 100% when the cells occupy the entire available surface of the support.

The different culture media indicated are well known by the person skilled in the art and can be prepared in the laboratory, usually from basic media, or can be acquired pre-prepared, ready for use, or in the absence of a certain ingredient.

Conventional culture media can be, for example, Dulbecco's Modified Eagle's Medium (DMEM), which is a modification of the Eagle's Basal Medium (EBM) with a concentration of amino acids, vitamins, and other additional components that is 4 times higher. There are several options of the DMEM medium, with different glucose concentrations, with or without L-glutamine and/or sodium pyruvate; and it often requires supplementing with serum.

Osteogenic culture media often contain dexamethasone, ascorbic acid or L-ascorbic acid phosphate, and β-glycerophosphate or sodium β-glycerophosphate, in different concentrations.

In this specification, blocking reagent is understood to mean the reagent, element, compound, molecule, and/or modified molecule which is capable of suppressing EphB3-mediated forward signaling and can be selected from the group consisting of: antibodies, EphB3-blocking peptides or fusion proteins, EphB3 gene expression-blocking interfering RNA (iRNA) and/or shRNA, edition of the gene encoding EphB3 by means of the CRISPR/CAS technique, or combinations thereof.

Among the anti-EphB3 antibodies, polyclonal or monoclonal antibodies can be selected. Blocking peptides are small protein molecules artificially designed for binding specifically to different EphB3 receptor activation domains and for preventing the binding of the corresponding ephrin. In terms of fusion proteins, they must be formed by the EphB3 receptor, to which another protein domain has been attached, such as the constant fraction (Fc) of immunoglobulin G (IgG), and prevent signaling through EphB3. The silencing method using iRNA is based on selecting and synthesizing a double-stranded RNA (dsRNA) which induces the degeneration of a messenger RNA to which it attaches by homology. In this particular case, the dsRNA has the sequence homologous with the messenger RNA for the EphB3 receptor. Similarly, short hairpin RNAs (shRNAs) are artificial RNA sequences that also generate iRNA to prevent the expression of the selected gene, but they must be introduced in cells by means of plasmids or viral vectors. In this way, the expression thereof is continuous, where gene expression can be blocked for a longer time. In the case of the present invention, the sequence of these shRNAs must be homologous with the sequence of the EphB3 gene. The CRISPR-CAS method also seeks to specifically silence the expression of EphB3, for which a guide complementary to the DNA sequence of the gene encoding EphB3 must be generated so that the system cleaves that sequence, causing that gene to not be expressed and the corresponding protein to not be synthesized.

In step 2), the osteogenic medium is preferably renewed with the blocking reagent at least every 7 days.

Throughout the method for enhancing the osteogenic differentiation of MSCs, the CO₂, temperature, and humidity conditions are those commonly used by the person skilled in the art: 5% CO₂, 37°C, and 100% humidity.

A second aspect of the invention relates to the use of the cells of the osteogenic lineage obtained by means of the method for enhancing osteogenic differentiation described above in a method for the treatment of bone diseases with bony tissue deficiency and/or bone fractures.

Cells of the osteogenic lineage include all existing cell types from MSCs to mature osteoblasts. According to different authors, these cell types have different names, the most common ones being: osteoprogenitor cells, preosteoblasts, osteoblasts, and mature osteoblasts.

A third aspect of the invention relates to an EphB3-receptor blocking reagent for use in the treatment of bone diseases with bony tissue deficiency and/or bone fractures.

The EphB3-receptor blocker can be selected from the group consisting of polyclonal or monoclonal antibodies, EphB3-blocking peptides or fusion proteins, EphB3 gene expression-blocking iRNA and/or shRNA, edition of the gene encoding EphB3 by means of the CRISPR/CAS technique, or combinations thereof.

A fourth aspect of the invention relates to a pharmaceutical composition including an EphB3-receptor blocking reagent for use in the treatment of bone diseases with bony tissue deficiency and/or bone fractures.

In this pharmaceutical composition, the EphB3-receptor blocker can be selected from the group consisting of polyclonal or monoclonal antibodies, EphB3-blocking peptides or fusion proteins, EphB3 gene expression-blocking iRNA and/or shRNA, edition of the gene encoding EphB3 by means of the CRISPR/CAS technique, or combinations thereof.

Likewise, the invention includes a pharmaceutical composition comprising cells of the osteogenic lineage obtained by means of the method described above, for use in the treatment of bone diseases with bony tissue deficiency and/or bone fractures

Another aspect of the invention relates to pharmaceutical compositions including cells of the osteogenic lineage obtained by means of the method described above and an EphB3-receptor blocking reagent.

Examples of the diseases with bony tissue deficiency for which the cells of the osteogenic lineage obtained by means of the method described above, the blocking reagents, and/or the pharmaceutical compositions of the invention can be used are: osteoporosis, both primary osteoporosis and osteoporosis due to the use of drugs which induce it (mainly immunosuppressive steroids), osteopenia, osteogenesis imperfecta, chronic kidney disease, hyperthyroidism, celiac disease, etc., preferably in mammals and, among others, in the human species.

The invention represents a novel contribution to methods for the osteogenic differentiation of mesenchymal stromal cells in mammals which significantly improves the capacity to obtain a large amount of bone, with application in the treatment of bone diseases and/or fractures.

### BRIEF DESCRIPTION OF THE DRAWINGS

To complement the description that is being made and for the purpose of helping to better understand the features of the invention, a set of drawings is attached as an integral part of said description in which the following is depicted in a non-limiting, illustrative character:
**Figure 1****.** Relative expression (RE) of pro-osteogenic transcription factors (RunX2, ALP, and Dlx5) in hMSCs from bone marrow which are cultured for 7 days in an osteogenic medium (OM) with different blockers at 5 and 10 µg/ml (* p-value<0.05, ** p-value<0.01 and *** p-value<0.005).
**Figure 2****.** Relative expression (RE) of pro-osteogenic transcription factors (RunX2, ALP, Dlx5, SP7, and MSX2) in hMSCs from bone marrow which are cultured for 7 days with different blockers at 5 µg/ml (* p-value<0.05, ** p-value<0.01 and *** p-value<0.005).
**Figure 3****.** Osteogenic differentiation quantification by alizarin red (AR) and cetylpyridinium chloride (CPC) staining of hMSCs from bone marrow which are treated for 21 days with different blockers at 5 µg/ml (* p-value<0.05, ** p-value<0.01 and *** p-value<0.005).
**Figure 4****.** Relative expression (RE) of pro-osteogenic transcription factors in the hTERT cell line transduced to overexpress EphB3. (* p-value<0.05 and ** p-value<0.01).
**Figure 5****.** Osteogenic differentiation quantification for 14 days by AR and CPC staining of the hTERT cell line transduced to overexpress EphB3 (*** p-value<0.005).
**Figure 6****.** Relative expression (RE) of pro-osteogenic transcription factors in MSCs from WT mice (unmodified) and EphB3-deficient mice (EphB3^{-/-}), cultured for 7 days (** p-value<0.01 and *** p-value<0.005).
**Figure 7****.** Osteogenic differentiation of murine MSCs, MSCs from WT mice and from EphB3⁻¹⁻ mice, after 21 days of culture. **A and B:** images of the cultures stained with AR (100X, scale = 200 µm). **C:** osteogenic differentiation quantification for 21 days by AR and CPC staining (*** p-value<0.005).
**Figure 8****.** Morphometric bone analysis by X-ray microtomography (microCT). **A:** images of the 3D reconstructions of the vertebrae of healthy control animals, ovariectomized animals, and animals treated with dexamethasone. **B and C:** bone volume (%BV/TV) and trabecula thickness (Tb.th) quantification (*** p-value<0.005) of the three groups of animals.

### PREFERRED EMBODIMENT OF THE INVENTION

The present invention is illustrated by means of the following examples that do not seek to limit the scope thereof.

### Example 1. MSC isolation and culture

Human MSCs were obtained both from bone marrow (Hospital Universitario de Valladolid) and from adipose tissue (Hospital Universitario Fundación Jiménez Diaz, Madrid) of different healthy donors and according to the Helsinki Declaration; furthermore, the Ethics Committee of both hospitals authorized the supply to conduct the study.

To achieve greater MSC enrichment, isolated cells were first cultured in a specific MesenCult^{™} Proliferation Kit culture medium (STEMCELL Technologies, France), which favors MSC proliferation in comparison with any other cell type present after the isolation.

16 ml of medium and 750,000 cells (5,000 cells/cm²) were placed in T150 flasks (culture support). The cultures were kept in an CO₂ incubator at a temperature of 37°C and saturating humidity conditions (100%). The culture medium was renewed at least once weekly.

When the cells reached 80%-90% confluence (confluence being understood to mean the degree at which the cells occupy the support where they are being cultured), they were lifted (detached) using a reagent to eliminate the attachments established between the cells and the culture surface; in this case, 6 ml of 0.025% trypsin (Hyclone, USA) diluted in DMEM culture medium (Lonza, Switzerland) were used. After 5 minutes of incubation at 37°C, the bottom of the flask was tapped softly to favor the detachment of the cells, and the reaction was stopped with DMEM + 10% FBS (Gibco, USA).

Once counted, suspended cells were passed to another T150 flask with 16 ml of conventional culture medium: DMEM (Lonza) + 10% fetal bovine serum (FBS (Gibco)), 1% antibiotic/antimycotic (Linus, Germany), 1% L-glutamine (Linus), and 1% sodium pyruvate (Sigma-Aldrich, USA), where the cells will become attached again and continue to proliferate (this is known as "passage"), increasing the number of MSCs in the culture. The MSCs used in all the described assays were in passage 3-6.

### Example 2. MSC characterization

MSCs in culture, in addition to their capacity to adhere to plastic and their fibroblastic morphology, are phenotypically characterized by flow cytometry to check the degree of purity of the culture. To that end, antibodies against surface molecules of MSCs were used. In that sense, human MSCs exhibited the CD73⁺ CD90⁺ CD105⁺ CD166⁺ CD14⁻CD34⁻ CD45⁻ HLA-DR⁻ phenotype and murine MSCs exhibited the Sca1⁺ CD29⁺ CD44⁺ CD73⁺ CD105⁺ CD106⁺ CD11b⁻ CD45⁻phenotype.

### Example 3. MSC differentiation

Once the MSCs have been characterized as described in Example 2, they were seeded in another new culture support, which is different based on the assay to be performed, at a density of 6,000 cells/cm² so that they quickly reached a confluence level not less than 90%. The MSCs were cultured in conventional medium, DMEM (Lonza) supplemented with 10% FBS (Gibco) and 1% L-glutamine (Linus), 1% sodium pyruvate, (Sigma-Aldrich), and 1% antibiotic/antimycotic (Linus). The culture medium was renewed at least one time weekly until the cells reaches 90% confluence.

Two types of differentiation assays which were carried out in different culture supports were performed: RT-qPCR, for which the MSCs were seeded in T25 flasks and 5 ml of conventional medium, and histological staining with alizarin red (AR) which was performed on P24 plates with 500 µl of conventional medium.

Upon reaching the desired confluence, the conventional culture medium was replaced with another osteogenic medium. In this example, MesenCult^{™} Osteogenic Differentiation Kit (STEMCELL Technologies) was used. The corresponding EphB3-blocking reagent was incorporated into this medium for enhancing the osteogenic differentiation of MSCs.

**Example 3.1.** Determination of the working concentration of the blockers using a polyclonal anti-EphB3 antibody (AF5667) and a fusion protein EphB3-Fc, at two different concentrations: 5 µg/ml and 10 µg/ml.

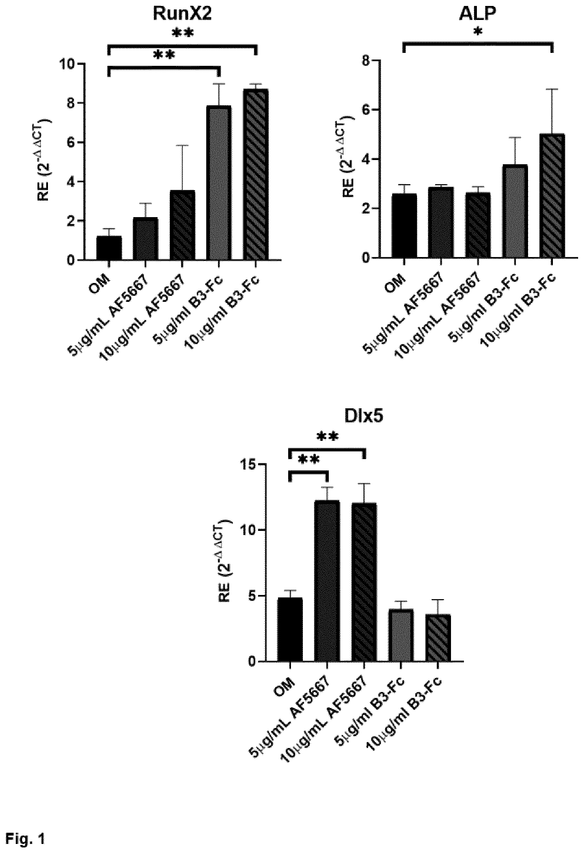

A polyclonal anti-EphB3 antibody (R&D Systems, USA) and a fusion protein EphB3-Fc (R&D Systems) were used as blocking reagents. Different concentrations (5 µg/ml and 10 µg/ml) of the antibody and the fusion protein in the osteogenic medium, that were kept for 7 days in T25 flasks with MSCs at 90% confluence in culture, were analyzed. After said time, the medium was removed and the cells lifted with trypsin (as described in Example 1), being frozen to extract their RNA later on. To that end, a total RNA extraction kit (Biotools, Spain) was used and, once quantified, 0.5 µg were reversed transcribed to cDNA/tube using a kit with a high-capacity reverse transcriptase (Applied Biosystems). A quantitative PCR, which allowed determining the amount of transcripts of the genes of interest that were present in the analyzed cells, was performed with that cDNA. The genes selected to perform this protocol encode transcription factors related to MSC bone differentiation: Runx2, ALP, and DIx5. In several treatments, an increase was observed in the expression of several of these differentiation genes, with respect to the control condition, similar in both concentrations, so it was decided to continue the rest of the analysis with 5 µg/mL of the blocking reagents (Figure 1).

**Example 3.2.** Genetic analysis of osteogenic differentiation after 7 days with several EphB3 blockers at 5 µg/ml: polyclonal anti-EphB3 antibody (AF5667), monoclonal anti-EphB3 antibody (MAB5667), monoclonal anti-EphB3 antibody (MAB56671), and fusion protein EphB3-Fc.

All the blocking reagents are sold by R&D Systems. In this case, they were used at a concentration of 5 µg/ml in osteogenic medium for 7 days for the genetic analysis of differentiation.

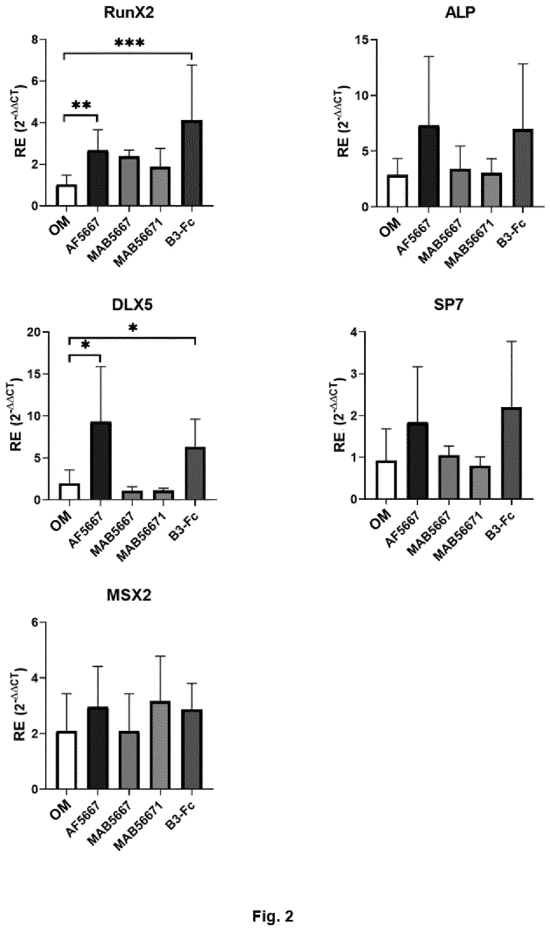

To analyze short-term differentiation, 150,000 cells were seeded in each T25 flask with 5 ml of supplemented DMEM culture medium as indicated in Example 1. After reaching 90% confluence, induction of osteogenic differentiation was started using MesenCult^{™} Osteogenic Differentiation Kit culture medium (STEMCELL Technologies) and each of the blocking reagents to be analyzed, at a concentration of 5 µg/ml, which were kept for 7 days. The cells were then lifted with trypsin (as described above) and frozen to extract their RNA later on. To that end, the same protocol described in Example 3.1 was followed. The genes selected to perform this protocol encode transcription factors related to MSC bone differentiation: Runx2, ALP, Dlx5, SP7, and Msx2. In almost all the genes, a higher expression was observed in MSCs treated with polyclonal anti-EphB3 antibody (AF5667) and EphB3-Fc, when compared to the control condition in which only osteogenic medium was used (Figure 2).

**Example 3.3.** Histological analysis of osteogenic differentiation after 21 days with several EphB3 blockers at 5 µg/ml: polyclonal anti-EphB3 antibody (AF5667), monoclonal anti-EphB3 antibody (MAB5667), monoclonal anti-EphB3 antibody (MAB56671), and fusion protein EphB3-Fc.

To check the duration required for blocking EphB3 signaling, differentiation after 21 days of the culture in osteogenic medium was analyzed in different conditions. 20,000 cells/well were seeded in P24 culture plates with 500 µl of supplemented DMEM medium as described in Example 1, until reaching about 90% confluence. The different treatments mentioned above were then incorporated into the MSCs in the following manner:
- 7 days in osteogenic medium with each of the blocking reagents under study, at 5 µg/ml, followed by 14 more days only with osteogenic medium.
- 14 days in osteogenic medium with each of the blocking reagents under study, at 5 µg/ml, followed by 7 more days only with osteogenic medium.

In all cases, the corresponding culture media were each changed every 7 days. In addition to the different treatments, two controls were performed in these assays: one in which the MSCs were kept in supplemented DMEM, as indicated in Example 1, for 21 days and another in which the MSCs grew in osteogenic medium, but without adding any blockers.

After culturing for 21 days, cells were fixed for 1 hour at 4°C with 70% cold ethanol. Next, the wells were washed twice with PBS 1X and the working solution of alizarin red (1 g of solid Alizarin Network + 50 ml of distilled water and pH 4.1) was added, leaving it in the dark for 20 minutes at room temperature. Finally, they were washed twice with sterile distilled water.

To quantify the staining calorimetrically, cells were washed one more time with water and incubated with a 10% CPC solution (1 g of cetylpyridinium chloride in 10 ml of 10 mM sodium phosphate, pH 7), for 20 minutes under horizontal stirring and at room temperature. To end, 20 µl of each treatment were placed in a P96 ELISA plate, diluted with 180 µl of the 10% CPC solution, and quantified in an ELISA reader at 562 nm. The 10% CPC solution was used as a blank and the values obtained in each treatment were referenced to a standard curve plotted with known concentrations of alizarin red diluted in 10% CPC.

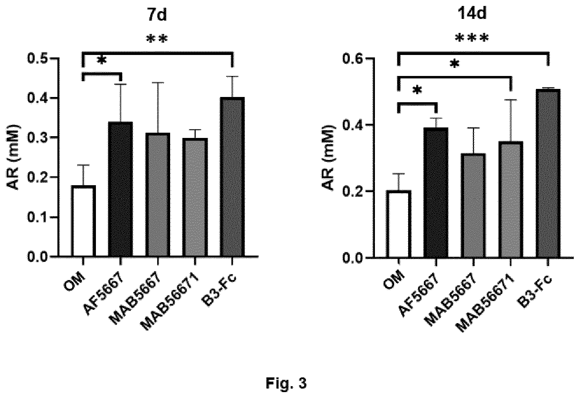

In all cases, an increase of the amount of alizarin red was observed upon blocking the EphB3 signal, being significant in the treatments with polyclonal antibody AF5667 and EphB3-Fc, both after 7 days and after 14 days, compared with the control condition of osteogenic medium (Figure 3).

### Example 4. Differentiation of hTERT cell line with EphB3 overexpression

In this case, a human MSC cell line (hTERT, supplied by Hospital Universitario de Valladolid), transduced with a lentiviral vector to overexpress the human EphB3 gene and two reporter genes, i.e., red fluorescent protein (RFP) gene and blasticidin resistance gene, was used, obtaining the hTERTMSC-EphB3 cell line. hTERT (hTertMSC-Control) transduced only with the reporter genes were used as control. To enrich the desired populations, blasticidin (5 ug/mL) was added to the culture medium used (supplemented DMEM described in Example 1) and, once expanded, RFP⁺ cells were isolated both from the control population and from the population that overexpressed EphB3, using a FACSAria separator cytometer (Becton Dickinson, CA, USA). Before starting the differentiation analysis, it was checked by means of RT-qPCR that the cells did indeed overexpress EphB3.

For the differentiation assays, 150,000 cells of each type (hTertMSC-EphB3 and hTertMSC-Control) were seeded in respective T25 flasks with supplemented DMEM and, once reaching 90% confluence, DMEM was replaced with MesenCult^{™} Osteogenic Differentiation Kit medium (STEMCELL Technologies), that was kept for 7 days. After this time, cells were lifted with trypsin (as described in Example 1) and frozen to extract their RNA later on.

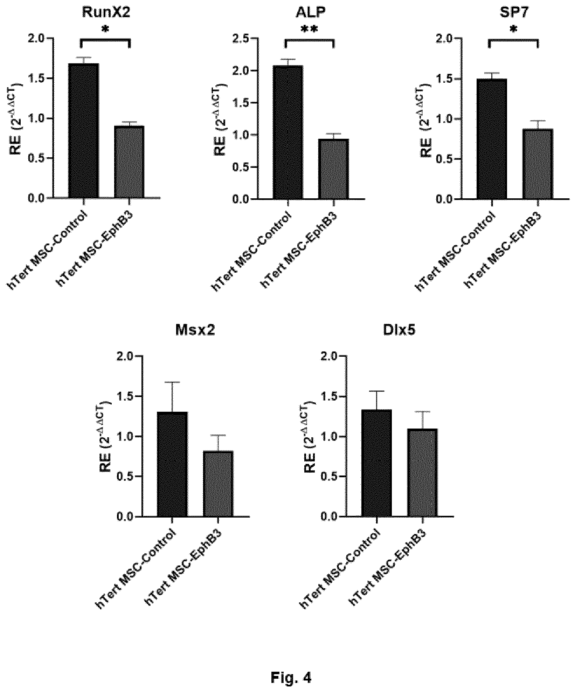

To perform RNA extraction, the same protocol described in Example 3.1 was followed. The genes selected to perform this protocol encode transcription factors related to MSC bone differentiation: RunX2, ALP, SP7, Msx2, and DIx5. It was observed that hTERT which overexpressed EphB3 (hTertMSC-EphB3) showed a lower expression of all the evaluated genes with pro-osteogenic activity, compared to the control condition, being significantly lower in RunX2, ALP, and SP7 (Figure 4).

Moreover, histological differentiation after 14 days of culture in osteogenic medium was also analyzed. To that end, 20,000 cells/well were seeded in P24 culture plates with 500 µl of supplemented DMEM medium as described in Example 1 until reaching about 90% confluence. Treatment was then started with MesenCult^{™} Osteogenic Differentiation Kit osteogenic medium (STEMCELL Technologies) which was kept for 14 days, changing it every 3-4 days.

After culturing for 14 days, cells were fixed for 1 hour at 4°C with 70% cold ethanol. Next, to assess osteogenesis, staining with alizarin red and CPC was performed, following the same method as that described in Example 3.3.

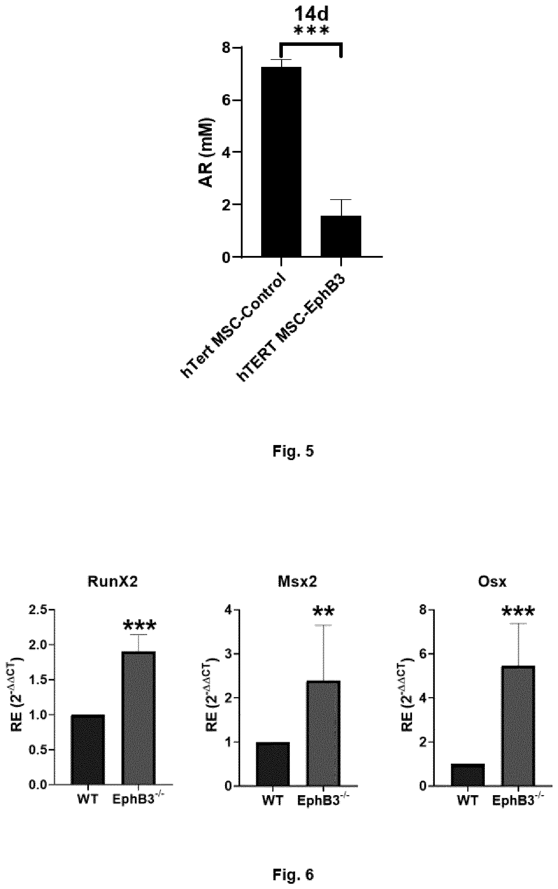

In this case, a significantly lower amount of alizarin red was obtained in hTERT which overexpressed EphB3 compared to control cells (Figure 5).

### Example 5. Genetic and histological analysis of osteogenic differentiation in EphB3 knockout mice.

MSCs originating from adipose tissue of wildtype (WT) mice and EphB3-deficient knockout mice (EphB3^{-/-}), which were isolated following the protocol described in Example 1, were analyzed. MSCs were placed in culture and subjected to repeated passages until their phenotype are verified by flow cytometry. Once the cells (between passages 3 and 6) have been prepared, they were seeded at a density of 6,000 cells/cm² in T25 flasks and P24 plates, with MesenCult^{™} Osteogenic Differentiation Kit medium (STEMCELL Technologies), for analysis by RT-qPCR (as described in Example 4) and by alizarin red staining (as described in Example 3.3), respectively. The MSCs in T25 were in culture for 7 days, whereas the MSCs for histological staining were in culture for 21 days, renewing the media once a week.

RT-qPCR showed a significant increase in the expression of several of the analyzed pro-osteogenic genes (RunX2, Msx2, and Osx; Figure 6), in EphB3⁻¹⁻ MSCs, with respect to WT cells.

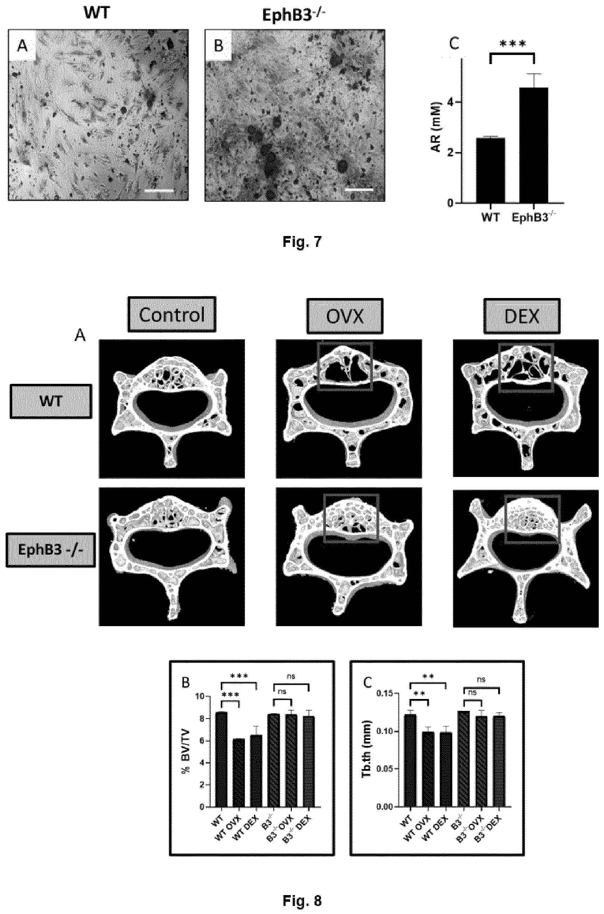

Clear evidence of increased accumulation of calcium phosphate in wells with EphB3^{-/-}cells, quantified by means of alizarin red staining (Figure 7) is also observed in histological staining.

### Example 6. In vivo assays in two murine models of osteoporosis

Two animal models of osteoporosis emulating the two most common causes of the onset of osteoporosis in humans, i.e., decreased estrogen levels and prolonged treatment with glucocorticoids, were used.

The first model is based on the loss of bony tissue which causes a drop in estrogen levels, due to increased osteoclastic activity and decreased osteoblast differentiation (something similar to what happens during menopause in humans). To achieve this hormone drop in mice, a bilateral ovariectomy (OVX) was performed. Briefly, two-month old female mice were anesthetized with a mixture of xylazine and ketamine, and two cuts were made on the skin and muscle to expose the ovaries and remove them with the help of a hot scalpel (to cauterize blood vessels) and a forceps. The incisions were then sutured and the mice were kept for 2 months until analysis.

The second model is based on the effect of glucocorticoids on bone remodeling process, since they increase osteoclast survival and activation, causing increased bone degradation. Two-month old female mice were used and treated with 2.5 mg/kg/day of dexamethasone (DEX) for 14 days, injected intraperitoneally, and they were analyzed the day after ending the treatment.

Both experimental methods were performed in WT and EphB3^{-/-} mice.

All the treated animals were sacrificed by cervical dislocation, extracting the left femur and the second lumbar spine, to be analyzed by means of X-ray microtomography (microCT) (Figure 8A).

In both disease models, it was determined that WT mice exhibited the expected osteoporotic phenotype, showing a significant decrease of both bone volume (BV/TV), calculated as the ration of bone volume (BV) and total volume (TV) of the analyzed section, and of trabecula thickness (Tb.Th) in femur and vertebra, which were quantified by means of the BoneJ software (GitHub) (Figures 8B and C).

However, EphB3^{-/-} mice did not exhibit differences between healthy controls and any of the animals with induced osteoporosis (OVX or DEX), in any of the parameters analyzed (Figures 8A, B, and C). This allows concluding that the lack of EphB3 in these mice prevents them from exhibiting the typical osteoporotic phenotype that is indeed observed in WT mice.

## Claims

1. A method for enhancing the osteogenic differentiation of mesenchymal stromal cells (MSCs) isolated and cultivated *in vitro* and obtaining cells of the osteogenic lineage, which includes the following steps:
1) after reaching 80-90% confluence between cells, replacing the culture medium used for culturing MSCs with an osteogenic medium and adding an EphB3-receptor blocking reagent;
2) incubating for at least 7 days.

2. The method for enhancing the osteogenic differentiation of MSCs according to claim 1, wherein the *in vitro* culture of isolated MSCs is performed in a culture medium which favors MSC proliferation.

3. The method for enhancing the osteogenic differentiation of MSCs according to any of the preceding claims, wherein step 1) is performed when the cells reach 90% confluence.

4. The method for enhancing the osteogenic differentiation of MSCs according to any of the preceding claims, wherein the osteogenic medium with the EphB3-receptor blocking reagent is renewed at least every 7 days.

5. The method for enhancing the osteogenic differentiation of MSCs according to any of the preceding claims, wherein the cells are from a mammal.

6. The method for enhancing the osteogenic differentiation of MSCs according to claim 5, wherein the mammal is a human.

7. MSC cells obtained according to the method defined in any of claims 1-6 for use in the treatment of bone diseases with bony tissue deficiency and/or bone fractures.

8. MSC cells obtained according to the method defined in any of claims 1-6 for use according to claim 7, wherein the bone diseases with bony tissue deficiency belong to the group comprising: primary osteoporosis, drug-induced osteoporosis, osteopenia, osteogenesis imperfecta, chronic kidney disease, hyperthyroidism, celiac disease.

9. An EphB3-receptor blocking reagent, element, compound, molecule, and/or modified molecule for use in the treatment of bone diseases with bony tissue deficiency and/or bone fractures.

10. The EphB3-receptor blocking reagent, compound, and/or molecule according to claim 9, selected from the group consisting of: polyclonal antibodies, monoclonal antibodies, fusion proteins, or combinations thereof.

11. The EphB3-receptor blocking reagent, compound, and/or molecule according to any of claims 9-10, wherein the bone diseases with bony tissue deficiency belong to the group comprising: primary osteoporosis, drug-induced osteoporosis, osteopenia, osteogenesis imperfecta, chronic kidney disease, hyperthyroidism, celiac disease.

12. The EphB3-receptor blocking reagent, compound, and/or molecule according to any of claims 9-11 for use in a mammal.

13. The EphB3-receptor blocking reagent, compound, and/or molecule according to claim 12, wherein the mammal is a human.

14. A pharmaceutical composition including an EphB3-receptor blocking reagent, element, compound, molecule, and/or modified molecule for use in the treatment of bone diseases with bony tissue deficiency and/or bone fractures.

15. The pharmaceutical composition according to claim 14, wherein the EphB3-receptor blocking reagent, element, compound, molecule, and/or modified molecule is selected from the group consisting of: polyclonal antibodies, monoclonal antibodies, fusion proteins, or combinations thereof.

16. A pharmaceutical composition including cells of the osteogenic lineage obtained according to the method defined in any of claims 1-6.

17. The pharmaceutical composition according to any of claims 14-15 including cells of the osteogenic lineage obtained according to the method defined in any of claims 1-6.

18. The pharmaceutical composition according to any of claims 14-17, wherein the bone diseases with bony tissue deficiency belong to the group comprising: primary osteoporosis, drug-induced osteoporosis, osteopenia, osteogenesis imperfecta, chronic kidney disease, hyperthyroidism, celiac disease.
